# EUROPEAN PATENT APPLICATION

(11) **EP 1 632 208 A1**
(43) Date of publication of application: **08.03.2006**
(21) Application number: 04745839.3
(22) Date of filing: 08.06.2004
(51) Int. Cl.: A61J 1/00, A61M 11/02

(54) **AEROSOL PREPARATION COMPRISING SEALED CONTAINER AND ENCLOSED THEREIN AEROSOL COMPOSITION CONTAINING MACROLIDE COMPOUND**

(30) Priority: 10.06.2003 JP 2003165161
(71) Applicant: Astellas Pharma Inc., Tokyo 103-8411 (JP)
(72) Inventor: YOSHIDA, Hiromitsu, Kyoto-shi, Kyoto 612-0806 (JP); ITO, Hideki c/o Astellas Pharma Inc., Chuo-ku, Tokyo 103-8411 (JP)
(74) Representative: Gille Hrabal Struck Neidlein Prop Roos
(86) International application number: PCT/JP2004/008270
(87) International publication number: WO 2004/110335

(57) **Abstract**

An aerosol preparation comprising an enclosure enclosing an aerosol composition containing a macrolide compound (e.g., tacrolimus) serving as an active ingredient has a problem such that the formulation of the aerosol composition is changed with the passage of time so as to reduee the ratio of the active ingredient in the aerosol composition because the macrolide compound as the active ingredient is partly adsorbed to a "gasket" of a valve part made of resinous material.

In an aerosol preparation of the invention comprising an aerosol composition containing a macrolide compound and an enclosure enclosing the aerosol composition, a valve part of the enclosure has a "gasket" made of at least one resinous material selected from butyl rubber, ethylene-propylene rubber, chloroprene rubber, polyethylene, polybutylene terephthalate, polyacetal, polyamide, polytetrafluoroethylene, polypropylene and thermoplastic elastomer, thereby solving the problem.

## Description

### TECHNICAL FIELD

The invention relates to an aerosol preparation comprising an enclosure enclosing an aerosol composition containing a macrolide compound.

### BACKGROUND ART

There are well-known conventional macrolide compounds such as rapamycin, tacrolimus and askomycin, which are generated by Streptomyces, analogues thereof, and derivatives thereof. The term "macrolide compound" is the generic name of compounds with 12 members or more, which belongs to macrolide lactones. Preferable examples of the macrolide compound are tricyclic compounds and pharmaceutically acceptable salts thereof.

Such tricyclic compounds, and rapamycin and its derivatives, have a similar basic structure, i.e., tricyclic macrolide structure, and at least one of the similar biological properties (for example, immunosuppressive activity).

On the other hand, a medical aerosol preparation (herein, combination of an aerosol composition and an enclosure enclosing the aerosol composition is referred to as an "aerosol preparation") is broadly used in the field of medicine as a drug delivery system, i.e., an inhaler, adapted to deliver a medicinally active substance in a finely divided form along with inspired air into the recipient's airway for the treatment of attacks of bronchial asthma, for instance.

An example of conventional aerosol composition used for the medical aerosol preparation contains the macrolide compound as an active ingredient, and an example of the macrolide compound is tacrolimus. This conventional aerosol composition may further contain lubricant consisting of medium-chain fatty acid triglyceride (a saturated fatty acid triglyceride [CH3(CH2)nCOOH; n = 4 to 10]), propellant consisting of liquefied hydrofluoroalkane, e.g., HFA-134a, and/or so on as occasion demands. Such an aerosol composition is disclosed in International Publication WO 97/10806, for example.

As disclosed in International Publication WO 99/43575, for instance, a metered dose inhaler (MDI) serves as an example of the enclosure for enclosing such an aerosol composition. The metered dose inhaler mainly comprises a vessel part, a cap part, and a valve part. The valve part is used for spraying out a dose of the aerosol composition from the vessel part, and the valve part has a structural portion functioning as a gasket (packing) in contact with the cap part, and/or interposed between the vessel part and the cap part, so as to prevent the aerosol composition from leaking out. Hereinafter and in attending claims, such a structural portion of the valve part having the above function is referred to as a "gasket".

### SUMMARY OF THE INVENTION

Resin is broadly used as material for constituting the "gasket" of the valve part of the metered dose inhaler. However, it has been found that the macrolide compound including the tacrolimus, when prepared for inhalation and enclosed in the metered dose inhaler, is partly adsorbed (or sorbed) to the resinous material made into the "gasket" of the valve part. This phenomenon suggests that the formulation of the aerosol composition enclosed and preserved in the metered dose inhaler is changed with the passage of time so that the ratio of active ingredient in the aerosol composition is reduced. Particularly, the reduction of the content of active ingredient by the adsorption thereof to the "gasket" of the valve part is serious in the case that the density of active ingredient in the aerosol composition when initially enclosed in the enclosure is low.

Considering the above situation, the present invention is intended to provide an aerosol preparation in which an aerosol composition containing a macrolide compound such as tacrolimus can be reserved for a long time without change of the formulation thereof with the passage of time.

The problem to be solved by the invention has been described as the above. Solution of the problems will now be described.

To solve the above-mentioned problem, what material is selected for making the "gasket" of the valve part of the enclosure is important. The material must adsorb no (strictly, very little) macrolide compound such as tacrolimus.

The material also requires such a certain "strength" and a certain "softness" as to function as the "gasket" of the valve part. The balance between strength and softness must be adjusted considering the structural location of the "gasket" in the valve part.

From this viewpoint, optimal materials for making a "gasket" of an aerosol preparation comprising an enclosure enclosing an aerosol composition containing a macrolide compound (such as tacrolimus) serving as an active ingredient have been sought. As a result, a solvable aerosol preparation is as follows:

An aerosol preparation comprises an aerosol composition containing a macrolide compound, and an enclosure enclosing the aerosol composition. The enclosure includes a valve part having a "gasket" made of at least one material selected from butyl rubber, ethylene-propylene rubber, chloroprene rubber, polyethylene, polybutylene terephthalate, polyacetal, polyamide, polytetrafluoroethylene, polypropylene and thermoplastic elastomer.

### BRIEF DESCRIPTION OF DRAWINGS

Fig. 1 is a sectional side view of a metered dose inhaler when it is not applied.
Fig. 2 is a sectional side view of the metered dose inhaler when it is applied.
Fig. 3 is a diagram showing a relation of adsorptivity of an active ingredient in an aerosol composition to each of resinous materials.
Fig. 4 is a diagram showing variation of a ratio of rest of an active ingredient in an aerosol composition with the passage of time when the aerosol preparation is kept in stationary.

### BEST MODE FOR CARRYING OUT THE INVENTION

An aerosol preparation of the invention will be detailed.

The following examples are listed up as preferable resinous materials for making the "gasket". Incidentally, the "resinous materials" in this application are defined as macromolecular materials such as rubbers, plastics and thermoplastic elastomers. Each of the following example resinous materials may have additives broadly used for prevention of degradation or improvement of properties, such as a processing-stabilization agent (example: Irgafos 168, Irgafos P-EPQ (the trademarks of Chiba Specialty Chemicals Hldg)), an ultraviolet absorber agent (example: Chimassorb 81, Tinuvin 213 (the trade marks of Chiba Specialty Chemicals Hldg), Sumisorb 400 (the trade mark of Sumitomo Chemical Co., Ltd.)) and an anti-oxidant (example: Sumilizer GA80 (the trade mark of Sumitomo Chemical Co., Ltd.), Irganox 245, Irganox B215 (the trade marks of Chiba Specialty Chemicals H1dg)), without departure from the scope of the invention.

Butyl Rubber: a butyl rubber containing 0.5 to 2.5 mole % degree of unsaturation (containing a 0.5 to 2.5 mole % isoprene), e.g., Code No. 540 of Valois, etc.

Ethylene-Propylene Rubber: an ethylene-propylene rubber containing a 15 to 50 mole % propylene, or an ethylene-propylene diene rubber (ethylene-propylene rubber polymerized with a little third component such as ENB, 1,4-hexadiene, dicyclopentadiene), e.g., Code No. 808 of Valois, etc.

Chloroprene Rubber: a sulfur-modified, non-sulfur-modified or highly chrystalline chloroprene rubber, e.g., Neoprene (the trademark of Du Pont), Code No. 210B of Valois, etc.

Polyethylene: a low-density, medium-density or high-density polyethylene, e.g., Hostalen (the trademark of Bassel Polyolefine), etc.

Polybutylene Terephthalate: a polybutylene terephthalate as a condensation polymer of thetramethylene-glycol (1,4-butanediol) combined with terephthalic acid or dimethyl terephthalate, e.g., Valox (the trademark of General Electric Plastics), etc.

Polyacetal: a homopolymeric polyacetal (polyoxymethylene) or a copolymeric polyacetal, e.g., Delrin (the trademark of Du Pont), Kematal (the trademark of Ticona), etc.

Polyamide: nylon 6, nylon 12, nylon 11, nylon 610, nylon 612 or nylon 66, e.g., Zytel (the trademark of Du Pont), etc.

Polytetrafluoroethylene: Code No. 8070 of Nikko Shokai, etc.

Polypropylene: PP1013H1 of ExxonMobil, etc.

Thermoplastic Elastomer: at least one rubber as a soft segment selected from a rubber group consisting of butyl rubber, ethylene propylene rubber and chloroprene rubber, mixed with at least one plastic as a hard segment selected from a plastic group of polyethylene, polybutylene terephthalate, polyacetal, polyamide, polytetrafluoroethylene and polypropylene, e.g., Code No. S8501 of Valois, etc.

Of the above resinous materials for making the "gasket", further preferable resinous materials are as follows:
1) butyl rubbers
2) ethylene-propylene rubbers
3) chloroprene rubbers
4) a polyolefin thermoplastic elastomer, which is at least one rubber as a soft segment selected from a rubber group of butyl rubber, ethylene-propylene rubber and chloroprene rubber, mixed with at least one plastic as a hard segment selected from a plastic group of polyethylene, polybutylene terephthalate, polyacetal, polyamide, polytetrafluoroethylene and polypropylene (further preferably, a polyolefin thermoplastic elastomer as a mixture of butyl rubber and polyethylene (the mixture (weight) ratio is between about 2:3 to about 3:2))

In addition to the above thermoplastic elastomer, a polystyrene thermoplastic elastomer (e.g., a mixture of polystyrene and polybutadiene, or a mixture of polystyrene and polyisoprene), a 1,2-polybutadiene thermoplastic elastomer (e.g., a mixture of syndiotactic 1,2-polybutadien and amorphous 1,2-polybutadien), a polyolefin thermoplastic elastomer (e.g., a mixture of polyethylene or polypropylene and ethylene-butene rubber), a polyurethane thermoplastic elastomer (a mixture of polyurethane and polyester, or a mixture of polyurethane and polyether), a polyester thermoplastic elastomer (e.g., a mixture of polyester and polyether), a polyamide thermoplastic elastomer (e.g., a mixture of polyamide and polyester, or a mixture of polyamide and polyether), a chlorinated polyethylene thermoplastic elastomer (e.g., a mixture of block type chlorinated polyethylene and random chlorinated polyethylene), a polyvinyl chloride thermoplastic elastomer (e.g., a mixture of polyvinyl chloride and amorphous polyvinyl chloride, or a mixture of polyvinyl chloride and acrylonitrile-butadiene rubber), or a polyfluorocarbon thermoplastic elastomer (e.g., a mixture of fluorocarbon resin and fluororubber) may serve as the resinous material for making the "gasket", if it adsorbs no (strictly, very little) macrolide compound.

As mentioned above, the resinous material for making the "gasket" must be selected so as to ensure an adequate balance between the certain strength and the certain softness. From this viewpoint, the resinous material must be selected considering the structural location of the "gasket" in the valve part.

Generally, the "gasket" constituting the valve part comprises the following three portions (not limited to this structure):
1) a gasket portion for ensuring airtightness of an inside (enclosed by a vessel part and a cap part) of the enclosure, namely, a neck gasket portion;
2) a gasket portion contacting a slide member so as to airtightly isolate the inside of the enclosure from a metering chamber, namely, a first gasket portion; and
3) a gasket portion contacting the slide member so as to airtightly isolate the metering chamber from the outside of the enclosure, namely, a second gasket portion.

The metering chamber is a structural portion of the valve part into which a fixed volume of the aerosol composition is taken out from the inside of the enclosure. The slide member is a structural portion of the valve part, which slides toward the inside of the enclosure so as to bring the metering chamber into communication with the inside of the enclosure, and which slides outward from the enclosure so as to bring the metering chamber into communication with an outside of the enclosure and to spray out the fixed volume of the aerosol composition.

In the present application, the above three gasket portions are referred to as the neck gasket portion, the first gasket portion and the second gasket portion, respectively.

The neck gasket portion for ensuring the airtightness of an enclosing chamber serving as the inside of the enclosure requires some degree of softness (plasticity or elasticity), and requires adhesion to tightly contact the vessel part, the cap part, or another portion of the valve part.

Also, the neck gasket portion having a large area in touch with the aerosol composition in the enclosing chamber is especially required to adsorb very little amount of the macrolide compound.

The first and second gasket portions airtightly and reciprocally slides against the slide member hundreds of times (depending on the filled volume) before the aerosol composition filled in the enclosure is completely used.

Therefore, the softness of the first and second gasket portions is required to be higher than that of the neck gasket portion.

Of course, the amount of the aerosol composition adsorbed to the first and second gasket portions is requested to be as little as possible. However, the first and second gasket portions, which are smaller than the neck gasket portion, have small surface areas in touch with the aerosol composition filled in the enclosure. It is guessed that the macrolide compound is adsorbed to every surface of the valve part in touch with the aerosol composition. Therefore, the material for making the first and second gasket portions is not required to adsorb so little amount of the macrolide compound as the material for making the neck gasket portion.

Thus, the material for making the first and second gasket portions can be selected attaching importance to the high softness.

In consideration of the above, preferable resinous material for making each of the gasket portions of the valve part are listed up as follows:

For the Neck Gasket Portion: a butyl rubber, e.g., Code No. 540 of Valois; or a thermoplastic elastomer, preferably, a mixture of butyl rubber and polyethylene (further preferably, the mixture (weight) ratio is set between about 2:3 to about 3:2), e.g., Code No. S8501 of Valois

For the First and Second Gasket Portions: a butyl rubber, e.g., Code No. 540 of Valois; an ethylene-propylene rubber, e.g., Code No. 808 of Valois; or a chloroprene rubber, e.g., Neoprene (the trade mark of Du Pont) or Code No. 210B of Valois (especially preferably, the chloroprene rubber, e.g., Code No. 210B of Valois)

Especially preferably, considering that the adsorption of the macrolide compound such as the tacrolimus to the resinous material should be restricted within a desirable range, the resinous material selected for making the "gasket" has such a characteristic that, just after the resinous material having 39mm² surface area has been soaked in the 10ml aerosol composition having the 0.025wt% macrolide compound for a month under a condition of 75% relative humidity and 40°C, the amount of the remaining macrolide compound in the aerosol composition is not less than 80% of the amount of the initial macrolide compound before the soaking.

The term "macrolide compound" is the generic name of compounds with 12 members or more, which belongs to macrolide lactones. Especially preferable examples of the macrolide compound are tacrolimus, a hydrate thereof and salts thereof.

An example of the "aerosol composition containing a macrolide compound" contains an active ingredient consisting of the macrolide compound (preferably, tacrolimus), and a propellant consisting of a liquefied hydrofluoroalkane. The aerosol composition may further contain a medium-chain fatty acid triglyceride serving as a lubricant. Examples of the medium-chain fatty acid triglyceride are such commercial products as Miglyol (the trademark of Sasol) 812, Panacete (the trademark of NOF corporation) 810, Coconard (the trademark of Kao corporation), Miritol (the trademark of Hankel-Hakusui) GM, ODO (the trademark of the Nisshin Oil Mills, Ltd.), etc.

Examples of the "liquefied hydrofluoroalkane" are HFA-134a, HFA-227, etc. One of them may be selected, or more than one may be selected to be mixed together. If the aerosol composition is prepared with the liquefied hydrofluoroalkane, the content of the tacrolimus solvable in the liquefied hydrofluoroalkane is not more than about ().15wt%.

The "aerosol composition containing a macrolide compound" can be prepared by the method normally used in the corresponding technological field.

A common valve used in the corresponding technological field can be used as the "valve part". Preferably, a valve part of a metered dose inhaler serves as the valve part.

Preferably, the "valve part" includes a metering chamber member, a housing and a protection ring to be discussed later in addition to the above-mentioned "gasket" and slide member.

ln the "aerosol preparation comprising an enclosure enclosing an aerosol composition containing a macrolide compound" according to the present invention, structural parts other than the above-mentioned parts can be prepared in an ordinary way used by a skilled person in the corresponding technological field.

Preferable embodiments of the present invention will be described as follows (the present invention is not limited to the following embodiments):

Preferably, the macrolide compound of the present invention is tacrolimus or hydrated tacrolimus.

Further preferably, the content of the tacrolimus or hydrated tacrolimus in the aerosol composition is not more than 0.15wt%.

Preferably, the aerosol composition of the present invention contains a liquefied hydrofluoroalkane.

Further preferably, the liquefied hydrofluoroalkane is one of HFA-134a and HFA-227, or a mixture of the two.

Preferably, the aerosol composition of the present invention contains a medium-chain fatty acid triglyceride.

Preferably, the enclosure of the present invention is a metered dose inhaler.

Preferably, material made into the "gasket" of the present invention is made of at least one resinous material selected from butyl rubber, ethylene-propylene rubber, chloroprene rubber and thermoplastic elastomer.

Preferably, the selected at least one resinous material made into the "gasket" of the present invention is the thermoplastic elastomer, which is at least one rubber selected from a rubber group consisting of butyl rubber, ethylene-propylene rubber and chloroprene rubber, mixed with at least one plastic selected from a plastic group of polyethylene, polybutylene terephthalate, polyacetal, polyamide, polytetrafluoroethylene and polypropylene.

Further preferably, the thermoplastic elastomer is a mixture of butyl rubber and polyethylene.

Preferably, the selected at least one resinous material made into the "gasket" of the present invention has such a characteristic that, just after the resinous material having 39mm² surface area has been soaked in the 10ml aerosol composition having the 0.025wt% macrolide compound for a month under a condition of 75% relative humidity and 40°C , the amount of the remaining macrolide compound in the aerosol composition is not less than 80% of the amount of the initial macrolide compound before the soaking.

Preferably, the "gasket" of the present invention comprises the neck gasket portion for ensuring airtightness of the enclosure, wherein the neck gasket portion is made of at least one resinous material selected from butyl rubber, ethylene-propylene rubber, chloroprene rubber, polyethylene, polybutylene terephthalate, polyacetal, polyamide, polytetrafluoroethylene, polypropylene and thermoplastic elastomer.

Further preferably, the selected at least one resinous material made into the neck gasket portion is the butyl rubber or the thermoplastic elastomer.

Preferably, the material made into the neck gasket portion of the present invention is a thermoplastic elastomer which is at least one rubber selected from a rubber group consisting of butyl rubber, ethylene-propylene rubber and chloroprene rubber, mixed with at least one plastic selected from a plastic group consisting of polyethylene, polybutylene terephthalate, polyacetal, polyamide, polytetrafluoroethylene and polypropylene.

Further preferably, the thermoplastic elastomer made into the neck gasket portion is a mixture of butyl rubber and polyethylene.

Preferably, the selected at least one resinous material made into the neck gasket portion of the present invention has such a characteristic that, just after the resinous material having 39mm² surface area has been soaked in the 10ml aerosol composition having the 0.025wt% macrolide compound for a month under a condition of 75% relative humidity and 40°C, the amount of the remaining macrolide compound in the aerosol composition is not less than 80% of the amount of the initial macrolide compound before the soaking.

Preferably, the valve part of the present invention is provided with a metering chamber, into which a fixed volume of the aerosol composition is taken out from the inside of the enclosure, and a slide member. The slide member slides toward the inside of the enclosure so as to bring the metering chamber into communication with the inside of the enclosure. The slide member slides outward from the enclosure so as to bring the metering chamber into communication with an outside of the enclosure and to spray out the fixed amount of the aerosol composition. The "gasket" of the valve part includes the neck gasket portion for ensuring airtightness of the enclosure, the first gasket portion contacting the slide member so as to airtightly isolate the inside of the enclosure from the metering chamber, and the second gasket portion contacting the slide member so as to airtightly isolate the metering chamber from the outside of the enclosure. Each of the neck gasket portion, the first gasket portion and the second gasket portion is made of at least one resinous material selected from butyl rubber, ethylene-propylene rubber, chloroprene rubber, polyethylene, polybutylene terephthalate, polyacetal, polyamide, polytetrafluoroethylene, polypropylene and thermoplastic elastomer.

Preferably, according to the present invention, the neck gasket portion is made of thermoplastic elastomer which is at least one rubber selected from a rubber group consisting of butyl rubber, ethylene-propylene rubber and chloroprene rubber, mixed with at least one plastic selected from a plastic group consisting of polyethylene, polybutylene terephthalate, polyacetal, polyamide, polytetrafluoroethylene and polypropylene, and each of the first and second gasket portions is made of butyl rubber, ethylene-propylene rubber or chloroprene rubber.

Further preferably, according to the present invention, the neck gasket portion is made of thermoplastic elastomer which is a mixture of butyl rubber and polyethylene, and the first and second gasket portions are made of chloroprene rubber.

Preferably, according to the present invention, the selected at least one resinous material made into each of the neck gasket portion, the first gasket portion and the second gasket portion has such a characteristic that, just after the resinous material having 39mm² surface area has been soaked in the 10ml aerosol composition having the 0.025wt% macrolide compound for a month under a condition of 75% relative humidity and 40°C, the amount of the remaining macrolide compound in the aerosol composition is not less than 80% of the amount of the initial macrolide compound before the soaking.

Preferably, the valve part of the present invention is provided with a metering chamber member, a housing and a slide member. If at least one of the metering chamber member, the housing and the slide member is made of resinous material, the resinous material is at least one selected from a plastic group consisting of polyethylene, polybutylene terephthalate, polyacetal, polyamide, polytetrafluoroethylene and polypropylene.

Further preferably, the selected resinous material is polybutylene terephthalate.

Further preferably, the selected at least one resinous material has such a characteristic that, just after the resinous material having 39mm² surface area has been soaked in the 10ml aerosol composition having the 0.025wt% macrolide compound for a month under a condition of 75% relative humidity and 40°C, the amount of the remaining macrolide compound in the aerosol composition is not less than 80% of the amount of the initial macrolide compound before the soaking.

Preferably, the valve part of the present invention is provided with a protection ring made of at least one resinous material selected from a plastic group consisting of polyethylene, polybutylene terephthalate, polyacetal, polyamide, polytetrafluoroethylene and polypropylene.

Further preferably, the selected resinous material made into the protection ring is polyethylene or polyamide.

Further preferably, the selected at least one resinous material made into the protection ring has such a characteristic that, just after the resinous material having 39mm² surface area has been soaked in the 10ml aerosol composition having the 0.025wt% macrolide compound for a month under a condition of 75% relative humidity and 40°C, the amount of the remaining macrolide compound in the aerosol composition is not less than 80% of the amount of the initial macrolide compound before the soaking.

The aerosol preparation comprising an aerosol composition containing a macrolide compound and an enclosure enclosing the aerosol composition according to the present invention has a prolonged pharmaceutical quality assurance such as to preserve the formulation of the aerosol composition therein from change with time.

The following embodiment of the aerosol preparation and various empiric test results will be detailed so as to confirm that the above effect is assured by optimal selection of material for making the "gasket" of the valve part of the enclosure.

### Embodiment of Aerosol Preparation Comprising Enclosure Enclosing Aerosol Composition Containing Macrolide Compound

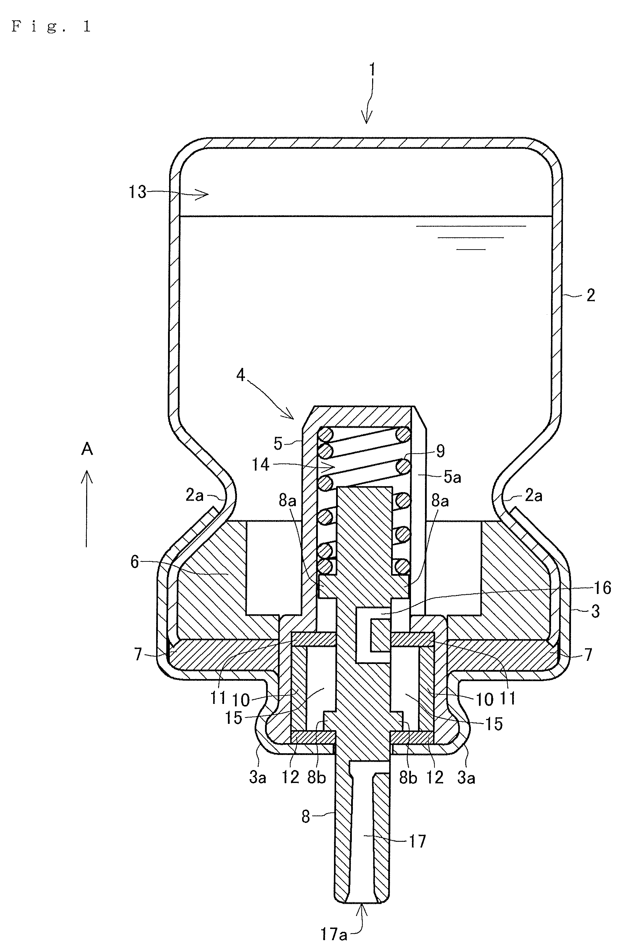

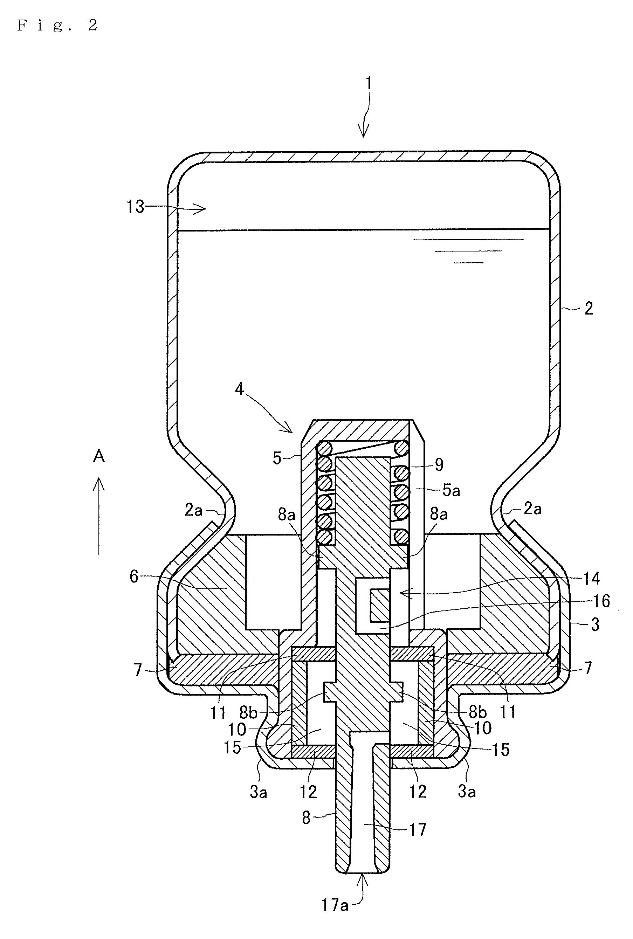

Referring to Figs 1 and 2, description will be given of a metered dose inhaler 1 serving as an example of an enclosure for constituting the "aerosol preparation comprising the enclosure enclosing an aerosol composition containing a macrolide compound" of the present invention, that is an enclosure applicable to preparation for inhalation.

Incidentally, the aerosol preparation according to the present invention is applicable to not only the following preparation for inhalation but also any other uses such as preparation of application for external use, medicine for nebulizer or medicine for internal use.

The following description will be given on the assumption for convenience that an arrow A in Fig. 1 points "upward".

As shown in Fig. 1, metered dose inhaler 1 mainly comprises a vessel part 2, a cap part 3, and a valve part 4.

Vessel part 2 for enclosing the aerosol composition is a main body of inhaler 1 formed into a substantially cylindrical shape having an open bottom surface and a closed top surface. Vessel part 2 is made of aluminum alloy in this present embodiment.

Cap part 3 is provided for hermetically sealing vessel part 1 after filled with the aerosol composition. Cap part 3 also serves as a fastener for fastening valve part 4 to vessel part 1.

Cap part 3, which is made of aluminum alloy similar to vessel part 2, is formed into a substantially cylindrical shape having an open bottom surface and a closed top surface. Cap part 3 is formed with a downwardly expanded portion 3a at its central bottom portion. Expanded portion 3a is crimped sideward so as to retain valve part 4 therein.

Expanded portion 3a has a center hole from which a slide member 8 serving as a component member of valve part 4 projects downward. A space enclosed by vessel part 2, cap part 3 and valve part 4, serving as an enclosing chamber 13, is filled with the aerosol composition containing tacrolimus, for example, as an active ingredient.

Valve part 4 serves as a gasket (packing) in contact with cap part 3 or interposed between vessel part 2 and cap part 3 so as to prevent the aerosol composition from leaking out, and is used for spraying a fixed volume of the aerosol composition outward from inhaler 1.

Valve part 4 in the present embodiment mainly comprises a housing 5, a protection ring 6, a neck gasket 7, slide member 8, a spring 9, a metering chamber member 10, a first gasket 11, a second gasket 12 and so on.

With respect to the shape of housing 5, two cylindrical upper and lower half portions having different diameters are layered. The lower half portion is diametrically larger than the upper half portion. The top surface of housing 5 is closed, and the bottom surface of housing 5 is opened. An inner portion of the upper half portion of housing 5 serves as a spring chamber 14, in which a spring 9 is disposed.

The upper half portion of housing 5 is provided at its peripheral surface with slits 5a,5a,5a for communicating spring chamber 14 therein to enclosing chamber 13 serving as a space filled with the aerosol composition.

Protection ring 6 is a member for supporting vessel part 2, i.e., for preventing vessel part 2 from being plastically pressed inwardly when cap part 3 fitted on vessel part 2 is plastically deformed so as to hermetically seal the aerosol composition. Protection ring 6, which is substantially ring-shaped in this embodiment, and neck gasket 7 are externally provided on the upper half portion of housing 5.

Neck gasket 7 for ensuring the airtightness of metered dose inhaler 1 is a substantially discoid member having a center hole in the present embodiment.

When the upper peripheral edge of cap part 3 is deformed centripetally so as to fit an annular recess 2a formed on the peripheral side surface of vessel part 2, the bottom edge of vessel part 2 is pressed downward so that the lower surface of neck gasket 7 comes to tightly fit to the inner surface of the bottom wall of cap part 3, and the bottom edge portion of vessel part 2 comes to abut against the upper surface of neck gasket 7.

In this way, neck gasket 7 is elastically or plastically deformed so that the edge surface of neck gasket 7 surrounding the center hole tightly contacts the outer peripheral surface of the lower half portion of housing 5, thereby ensuring the airtightness of enclosing chamber 13 serving as the inside space of metered dose inhaler 1.

Substantially columnar slide member 8 is disposed in housing 5 so as to be slidable upward and downward (i.e., in a withdrawing direction to the inside of inhaler 1 and in a projecting-out direction to the outside of inhaler 1).

Slide member 8 is formed at intermediate portions of the side surface thereof with two radially expanded engaging portions 8a and 8b.

In this condition, spring 9 is disposed in a spring chamber 14 serving as the inside space of the upper half portion of housing 5, and engages to engaging portion 8a formed on the upper side surface of slide member 8 so as to bias slide member 8 downward (i.e., in the projecting-out direction to the outside of inhaler 1).

In the lower half portion of housing 5, first gasket 11 and second gasket 12 are disposed up and down with metering chamber member 10 therebetween. Slide member 8 vertically penetrates the lower half portion of housing 5. First gasket 11, second gasket 12, metering chamber member 10, the lower half portion of housing 5 and the outer peripheral surface of slide member 8 enclose a space serving as a metering chamber 15 into which a fixed volume of the aerosol composition is taken out from enclosing chamber 13 of metered dose inhaler 1.

First and second gaskets 11 and 12 of this embodiment are substantially discoid members having respective center holes. Slide member 8 slidably fittingly penetrates the center holes so that first and second gaskets 11 and 12 airtightly slidably abut against the outer peripheral surface of slide member 8.

Engaging portion 8b is formed on a side surface of an intermediate portion of slide member 8 in metering chamber 15 below engaging portion 8a.

Slide member 8 downwardly biased by spring 9 is kept in stationary so that engaging portion 8b abuts against the upper surface of second gasket 12 (facing upward to metering chamber 15), when inhaler 1 is not applied as shown in Fig. 1.

A connection passage 16 and a spraying passage 17 are bored in slide member 8. The lower portion of slide member 8 projects downward from the hole in expanded portion 3a of cap part 3 (i.e., to the outside of inhaler 1).

Connection passage 16 has an upper open end, which is constantly open at the outer peripheral surface of slide member 8 so as to face spring chamber 14 whether inhaler 1 is applied as shown in Fig. 2 (slide member 8 is slid upward) or not applied as shown in Fig. 1 (slide member 8 is disposed at the lowest slide position so as to make engaging portion 8b abut against the upper surface of second gasket 12).

Connection passage 16 has a lower open end, which is open at the outer peripheral surface of slide member 8 so as to face into spring chamber 14 when inhaler 1 is out of use as shown in Fig. 1, and to face into metering chamber 15 when inhaler 1 is applied as shown in Fig. 2.

Spraying passage 17 has an upper open end at the outer peripheral surface of slide member 8, which is exposed out of inhaler 1 when inhaler 1 is not applied as shown in Fig. 1, and faces into metering chamber 15 when inhaler 1 is applied as shown in Fig. 2.

Spraying passage 17 has a lower open end serving as a spraying nozzle hole 17a, which is open downward at the lower end of slide member 8.

The volume of metering chamber 15 is set substantially equal to each dose of the aerosol composition sprayed from inhaler 1.

Due to the above construction, in the non-applied condition of inhaler 1 where slide member 8 is disposed at the lower slide position, as shown in Fig. 1, metering chamber 15 is airtightly isolated from the outside of inhaler 1, however, metering chamber 15 communicates with enclosing chamber 13 serving as the inner space of inhaler 1 through connection passage 16. Namely, while inhaler 1 is not applied, the fixed volume of aerosol composition corresponding to its one dose is reserved in metering chamber 15.

In the applied condition of inhaler 1 as shown in Fig. 2 where slide member 8 is slid upward, metering chamber 15 is airtightly isolated from enclosing chamber 13, and communicates to the outside of inhaler 1 through spraying passage 17.

In this condition, the pressure of the aerosol composition in metering chamber 15 is substantially equaled to that of the aerosol composition in enclosing chamber 13 higher than the atmospheric pressure, whereby the dose of aerosol composition in metering chamber 15 is sprayed from spraying nozzle hole 17a to the outside of inhaler 1.

Housing 5 and slide member 8 constitute a structural body of valve part 4 and are biased by spring 9. Also, metering chamber member 10 is provided for supporting first and second gaskets 11 and 12 in stationary against sliding of slide member 8. Therefore, metering chamber member 10, housing 5 and slide member 8 require high strength.

It is guessed that the macrolide compound including the tacrolimus is adsorbed onto only a surface of a member in touch with the aerosol composition. Therefore, housing 5, slide member 8 and protection ring 6 having large areas in touch with the aerosol composition in enclosing chamber 13 serving as the inside space of inhaler 1 are especially requested to adsorb very little macrolide compound.

From this viewpoint, it is considered that metal or resin is provided as material for making metering chamber member 10, housing 5, protection ring 6 and slide member 8. If a resinous material is used, it is preferably selected from a plastic group consisting of polyethylene, polybutylene terephthalate, polyacetal, polyamide, polytetrafluoroethylene and polypropylene. Especially, the polybutylene terephthalate is preferable.

For example, the selected resinous material may have such a characteristic that, just after the resinous material having 39mm² surface area has been soaked in the 10ml aerosol composition having the 0.025wt% macrolide compound for a month under a condition of 75% relative humidity and 40°C, the amount of the remaining macrolide compound in the aerosol composition is not less than 80% of the amount of the initial macrolide compound before the soaking.

To constitute valve part 4 in metered dose inhaler 1 of this embodiment, the "gasket", i.e., neck gasket 7, first gasket 11 and second gasket 12 are made of any of butyl rubber, ethylene-propylene rubber, chloroprene rubber, polyethylene, polybutylene terephthalate, polyacetal, polyamide, polytetrafluoroethylene, polypropylene and thermoplastic elastomer. Housing 5, slide member 8, metering chamber member 10 and protection ring 6 are made of any of polybutylene terephthalate, polyacetal, polyamide, polytetrafluoroethylene and polypropylene. Spring 9 is made of iron material such as stainless steel. Parts of inhaler 1 including vessel part 2 and cap part 3 excluding valve part 4 are made of metal material such as aluminum alloy.

In addition to the above-mentioned metering chamber member 10, housing 5, slide member 8 and protection ring 6, the "gasket" of the valve part can also be made of selected resinous material having such a characteristic that, just after the resinous material having 39mm² surface area has been soaked in the 10ml aerosol composition having the 0.025wt% macrolide compound for a month under a condition of 75% relative humidity and 40°C, the amount of the remaining macrolide compound in the aerosol composition is not less than 80% of the amount of the initial macrolide compound before the soaking.

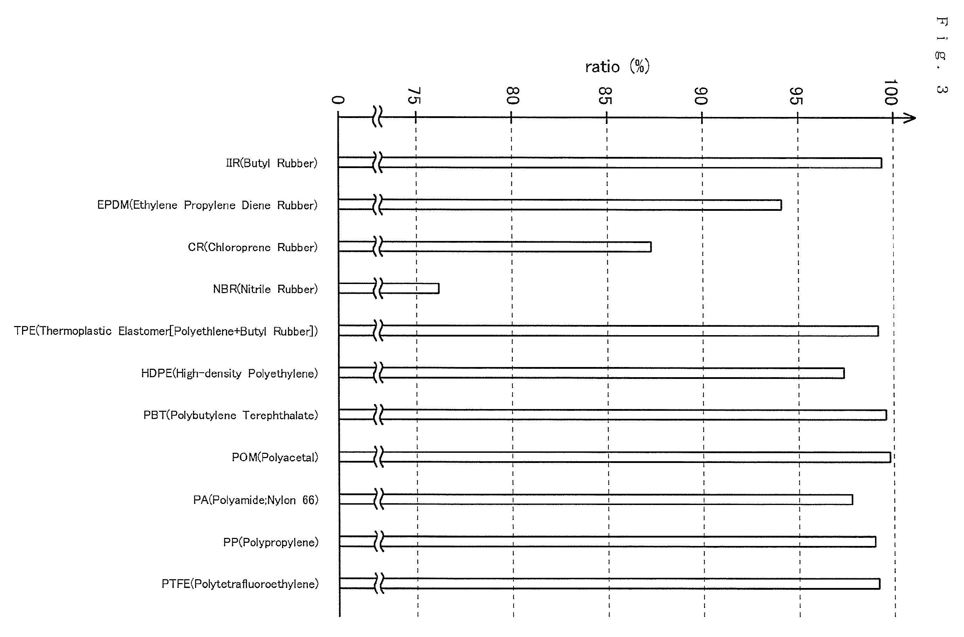

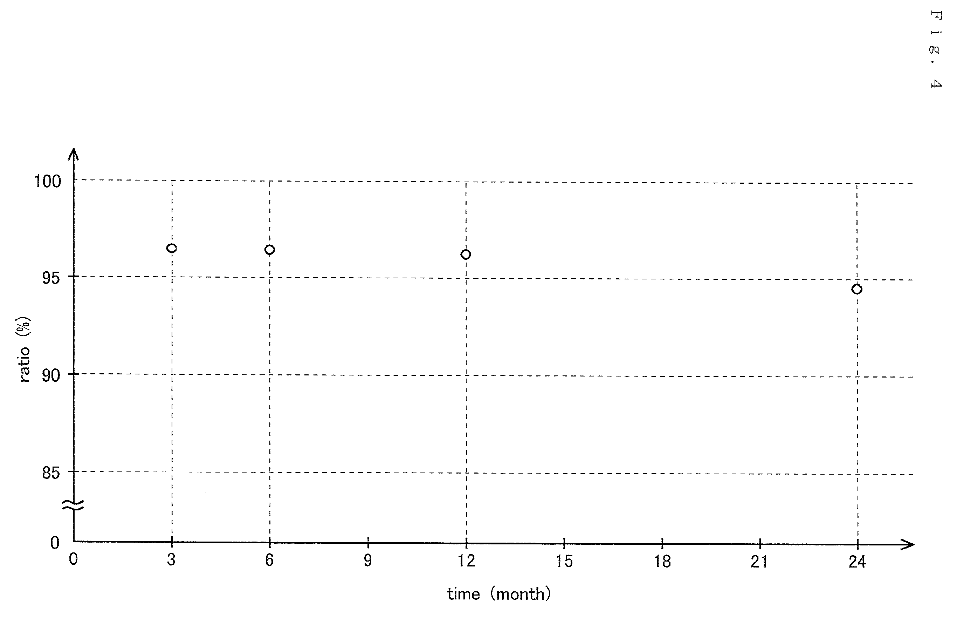

Referring to Figs. 3 and 4, results of testing absorption of tacrolimus as an example of the macxolide compound serving as an active ingredient in the aerosol composition according to the present invention will be described.

### Adsorptivity of Tacrolimus to Resinous Material Constituting Valve Part

Fig. 3 illustrates the adsorptivity of the active ingredient contained in the aerosol composition to each sample resinous material soaked in the aerosol composition.

The axis of ordinates designates the ratio of (remaining) active ingredient in the aerosol composition to the initial active ingredient therein when the percentage of the initial active ingredient contained in the aerosol composition is 100%.

The aerosol composition used in the test of Fig. 3 contains a 0.025wt% macrolide compound. To be concrete, the aerosol composition is a solution that contains 2.5mg tacrolimus serving as the active ingredient, 10ml liquefied hydrofluoroalkane (HFA-134a) serving as the propellant, and a little amount of lubricant normally used for metered dose inhalation.

In the test of Fig. 3, each of the sample resinous materials is formed into a circular shape having a 5mm diameter (a 39mm² surface area), similar to the form of first gasket 11. Each of the sample resinous materials is soaked in the aerosol composition solution under the condition of 75% relative humidity and 50°C for a month.

The soaked sample materials are BR (butyl rubber), EPDM (ethylene-propylene diene rubber, as a kind of ethylene-propylene rubber), CR (chloroprene rubber), PE (polyethylene, especially, high-density polyethylene), TPE (thermoplastic elastomer, that is a mixture of PE and BR in this test), PBT (polybutylene terephthalate), POM (polyoxymethylene as one of polyacetal), PA (polyamide), PTFE (polytetrafluoroethylene), PP (polypropylene), and NBR (nitrile rubber).

Commercial products as the respective sample resinous materials are as follows:
1) BR: Code No. 540 of Valois
2) EPDM: Code No. 808 of Valois
3) CR: Code No. 210B of Valois
4) High-density PE: the trademark Hostalen GC7260 of Bassel Polyolefine
5) TPE as a mixture of PE and BR: Code No. S8501 of Valois
6) PBT: the trademark Valox 312C-1001 of General Electric Plastics
7) POM: the trademark Kematal M270.14 of Ticona
8) PA (Nylon 66): the trademark Zytel E 101 LNC 10G of Du Pont
9) PTFE: Code No. 8070 of Nikko Shokai
10) PP: PP 1013H1 of ExxonMobil
11) NBR: Code No. 403 of Valois

The ratio of remaining active ingredient is an average of three soaked samples of each resinous material (n=3).

As understood from Fig. 3, when the nitrile rubber is soaked in the aerosol composition, the active ingredient in the aerosol composition is considerably reduced.

In other words, the nitrile rubber soaked in the aerosol composition adsorbs a considerably large amount of the active ingredient, i.e., tacrolimus.

The active ingredient in the aerosol composition is scarcely reduced when any of the butyl rubber, the ethylene-propylene rubber, the chloroprene rubber, the thermoplastic elastomer as the mixture of polyethylene and butyl rubber, the polyethylene, the polybutylene terephthalate, the polyacetal, the polyamide, the polytetrafluoroethylene and the polypropylene is soaked in the aerosol composition.

In other words, very little tacrolimus serving as the active ingredient is adsorbed to any of the butyl rubber, the ethylene-propylene rubber, the chloroprene rubber, the thermoplastic elastomer as the mixture of polyethylene and butyl rubber, the polyethylene, the polybutylene terephthalate, the polyacetal, the polyamide, the polytetrafluoroethylene and the polypropylene, which are soaked in the aerosol composition.

Consequently, the butyl rubber, the ethylene-propylene rubber, the chloroprene rubber, the polyethylene, the polybutylene terephthalate, the polyacetal, the polyamide, the polytetrafluoroethylene, the polypropylene and the thermoplastic elastomer are appropriate for material constituting valve part 4 of metered dose inhaler 1. The thermoplastic elastomer is, preferably, at least one rubber selected from a rubber group consisting of butyl rubber, ethylene-propylene rubber and chloroprene rubber, mixed with at least one plastic selected from a plastic group consisting of polyethylene, polybutylene terephthalate, polyacetal, polyamide, polytetrafluoroethylene and polypropylene.

Especially, the butyl rubber, the ethylene-propylene rubber, the chloroprene rubber and the thermoplastic elastomer (preferably, at least one rubber selected from a rubber group consisting of butyl rubber, ethylene-propylene rubber and chloroprene rubber, mixed with at least one plastic selected from a plastic group consisting of polyethylene, polybutylene terephthalate, polyacetal, polyamide, polytetrafluoroethylene and polypropylene) are preferable materials having the strength and softness required for the gasket.

The polyethylene, polybutylene terephthalate, polyacetal, polyamide, polytetrafluoroethylene and polypropylene are preferable materials for making the metering chamber member, the housing, the slide member and the protection ring in the valve part requiring high strength.

### Variation of Concentration of Tacrolimus in Aerosol Preparation

Fig. 4 illustrates variation of the ratio of rest of the active ingredient in the aerosol composition with the passage of time when a chloroprene rubber or a thermoplastic elastomer serves as a sample material for constituting the "gasket" of valve part 4. The axis of ordinates designates the ratio of (remaining) active ingredient in the aerosol composition to the initial active ingredient when the percentage of the initial active ingredient contained in the aerosol composition is 100%, and the axis of the axis of abscissas designates the passage of time.

The aerosol composition used in the test of Fig. 4 contains a 0.025wt% macrolide compound, similar to that used in the former test of adsorptivity of tacrolimus to materials for making the valve part, and it is enclosed in the enclosure so as to serve as the aerosol preparation (the preparation for inhalation shown in Fig. 1).

In the test of Fig. 4, housing 5, slide member 8 and metering chamber member 10 are made of the polybutylene terephthalate (Valox 312C-1001). First and second gaskets 11 and 12 are made of the chloroprene rubber (Code No. 210B of Valois), neck gasket 7 is made of the thermoplastic elastomer as the mixture of polyethylene and butyl rubber (Code No. S8501 of Valois). Protection ring 6 is made of the polyamide (Zytel E 101 LNC 10G). They are formed into the respective shapes and assembled together as shown in Fig. 1, thereby constituting metering dose inhaler 1. The aerosol composition is enclosed in inhaler 1 so as to make an aerosol preparation for inhalation. This aerosol preparation is kept in a condition of 60% relative humidity and 25°C equivalent to the ordinary room condition.

The ratio of remaining active ingredient at each period is an average of three soaked samples (n=3).

As understood from Fig. 4, just after twenty four months (two years) has passed, the remaining amount of active ingredient in the aerosol composition, in which the chloroprene rubber or the thermoplastic elastomer sampled as material for making the gasket of the valve part is soaked, is above 90% of the initial amount of active ingredient.

Consequently, when the chloroprene rubber or the thermoplastic elastomer is used as material for making the gasket of the valve part 4, the aerosol composition containing the tacrolimus can be substantially preserved for a long time from change of the formulation thereof with time.

As understood from the result of the test shown in Fig. 3, in the condition of 40°C and 75% relative humidity, the chloroprene rubber adsorbs a larger amount of tacrolimus than any other soaked sample materials, i.e., the butyl rubber, the ethylene-propylene rubber, the polyethylene, the polybutylene terephthalate, the polyacetal, the polyamide, the polytetrafluoroethylene, the polypropylene and the thermoplastic elastomer (the mixture of polyethylene and butyl rubber).

Consequently, it is suggested that, when any of the butyl rubber, the ethylene-propylene rubber, the polyethylene, the polybutylene terephthalate, the polyacetal, the polyamide, the polytetrafluoroethylene, the polypropylene and the thermoplastic elastomer (the mixture of polyethylene and butyl rubber) is selected as the material for making the "gasket" and soaked in the aerosol composition containing tacrolimus under the condition similar to that for the test of Fig. 4, the remaining amount of the active ingredient in the aerosol composition just after the passage of 24 months (two years) is significantly more than 90% of the initial amount of the active ingredient, similarly to the case of the chloroprene rubber soaked in the aerosol composition.

Consequently, when the "gasket" of valve part 4 is made of any material selected from the butyl rubber, the ethylene-propylene rubber, the polyethylene, the polybutylene terephthalate, the polyacetal, the polyamide, the polytetrafluoroethylene, the polypropylene and the thermoplastic elastomer (preferably, the mixture of polyethylene and butyl rubber), the aerosol composition containing the tacrolimus can be appropriately preserved for a long time with very little change of the content rate in the composition with the passage of time.

### INDUSTRIAL APPLICABILITY

The "aerosol preparation comprising an enclosure enclosing an aerosol composition containing a macrolide compound" according to the present invention is useful in the medical and pharmaceutical industrial field, for example.

## Claims

1. An aerosol preparation, comprising:
an aerosol composition containing a macrolide compound; and
an enclosure enclosing the aerosol composition, the enclosure including a valve part having a "gasket" made of at least one resinous material selected from butyl rubber, ethylene-propylene rubber, chloroprene rubber, polyethylene, polybutylene terephthalate, polyacetal, polyamide, polytetrafluoroethylene, polypropylene and thermoplastic elastomer.

2. The aerosol preparation according to claim 1, wherein the macrolide compound is tacrolimus or hydrated tacrolimus.

3. The aerosol preparation according to claim 2, wherein the content of the tacrolimus or the hydrated tacrolimus in the aerosol composition is not more than 0.15wt%.

4. The aerosol preparation according to any of claims 1 to 3, wherein the aerosol composition further contains a liquefied hydrofluoroalkane.

5. The aerosol preparation according to claim 4, wherein the liquefied hydrofluoroalkane is one of HFA-134a and HFA-227, or a mixture of them.

6. The aerosol preparation according to any of claims 1 to 5, wherein the aerosol composition further contains a medium-chain fatty acid triglyceride.

7. The aerosol preparation according to any of claims 1 to 6, wherein the enclosure is a metered dose inhaler.

8. The aerosol preparation according to any of claims 1 to 7, wherein the "gasket" is made of at least one resinous material selected from butyl rubber, ethylene-propylene rubber, chloroprene rubber and thermoplastic elastomer.

9. The aerosol preparation according to any of claims 1 to 8, wherein the selected at least one resinous material made into the "gasket" is the thermoplastic elastomer, which is at least one rubber selected from a rubber group consisting of butyl rubber, ethylene-propylene rubber and chloroprene rubber, mixed with at least one plastic selected from a plastic group consisting of polyethylene, polybutylene terephthalate, polyacetal, polyamide, polytetrafluoroethylene and polypropylene.

10. The aerosol preparation according to claim 9, wherein the thermoplastic elastomer is a mixture of butyl rubber and polyethylene.

11. The aerosol preparation according to any of claims 1 to 10, wherein the selected at least one resinous material made into the "gasket" has such a characteristic that, just after the resinous material having 39mm² surface area has been soaked in the 10ml aerosol composition having the 0.025wt% macrolide compound for a month under a condition of 75% relative humidity and 40°C, the amount of the remaining macrolide compound in the aerosol composition is not less than 80% of the amount of the initial macrolide compound before the soaking.

12. The aerosol preparation according to any of claims 1 to 10, the "gasket" comprising a neck gasket portion for ensuring airtightness of the enclosure, wherein the neck gasket portion is made of at least one resinous material selected from butyl rubber, ethylene-propylene rubber, chloroprene rubber, polyethylene, polybutylene terephthalate, polyacetal, polyamide, polytetrafluoroethylene, polypropylene and thermoplastic elastomer.

13. The aerosol preparation according to claim 12, wherein the selected at least one resinous material made into the neck gasket portion is the butyl rubber or the thermoplastic elastomer.

14. The aerosol preparation according to claim 13, wherein the thermoplastic elastomer made into the neck gasket portion is at least one rubber selected from a rubber group consisting of butyl rubber, ethylene-propylene rubber and chloroprene rubber, mixed with at least one plastic selected from a plastic group consisting of polyethylene, polybutylene terephthalate, polyacetal, polyamide, polytetrafluoroethylene and polypropylene.

15. The aerosol preparation according to claim 14, wherein the thermoplastic elastomer made into the neck gasket portion is a mixture of butyl rubber and polyethylene.

16. The aerosol preparation according to any of claims 12 to 15, wherein the selected at least one resinous material made into the neck gasket portion has such a characteristic that, just after the resinous material having 39mm² surface area has been soaked in the 10ml aerosol composition having the 0.025wt% macrolide compound for a month under a condition of 75% relative humidity and 40°C, the amount of the remaining macrolide compound in the aerosol composition is not less than 80% of the amount of the initial macrolide compound before the soaking.

17. The aerosol preparation according to any of claims 1 to 10, the valve part of the enclosure further comprising:
a metering chamber into which a fixed volume of the aerosol composition is taken out from the inside of the enclosure; and
a slide member, wherein the slide member slides toward the inside of the enclosure so as to bring the metering chamber into communication with an inside of the enclosure, and wherein the slide member slides outward from the enclosure so as to bring the metering chamber into communication with an outside of the enclosure and to spray out the fixed volume of the aerosol composition from the metering chamber, the "gasket" of the valve part including:
a neck gasket portion for ensuring airtightness of the enclosure;
a first gasket portion contacting the slide member so as to airtightly isolate the inside of the enclosure from the metering chamber; and
a second gasket portion contacting the slide member so as to airtightly isolate the metering chamber from the outside of the enclosure,
wherein each of the neck gasket portion, the first gasket portion and the second gasket portion is made of at least one resinous material selected from butyl rubber, ethylene-propylene rubber, chloroprene rubber, polyethylene, polybutylene terephthalate, polyacetal, polyamide, polytetrafluoroethylene, polypropylene and thermoplastic elastomer.

18. The aerosol preparation according to claim 17, wherein the neck gasket portion is made of thermoplastic elastomer which is at least one rubber selected from a rubber group consisting of butyl rubber, ethylene-propylene rubber and chloroprene rubber, mixed with at least one plastic selected from a plastic group consisting of polyethylene, polybutylene terephthalate, polyacetal, polyamide, polytetrafluoroethylene and polypropylene, and wherein each of the first and second gasket portions is made of butyl rubber, ethylene-propylene rubber or chloroprene rubber.

19. The aerosol preparation according to claim 18, wherein the neck gasket portion is made of thermoplastic elastomer which is a mixture of butyl rubber and polyethylene, and wherein each of the first and second gasket portions is made of chloroprene rubber.

20. The aerosol preparation according to any of claims 17 to 19, wherein the selected at least one resinous material made into each of the neck gasket portion, the first gasket portion and the second gasket portion has such a characteristic that, just after the resinous material having 39mm² surface area has been soaked in the 10ml acrosol composition having the 0.025wt% macrolide compound for a month under a condition of 75% relative humidity and 40°C, the amount of the remaining macrolide compound in the aerosol composition is not less than 80% of the amount of the initial macrolide compound before the soaking.

21. The aerosol preparation according to any of claims 1 to 20, the valve part further comprising a metering chamber member, a slide member and a housing,
wherein at least one of the metering chamber member, the slide member and the housing is made of at least one resinous material selected from a plastic group including polyethylene, polybutylene terephthalate, polyacetal, polyamide, polytetrafluoroethylene and polypropylene.

22. The aerosol preparation according to claim 21, wherein the at least one of the metering chamber member, the slide member and the housing is made of polybutylene terephthalate.

23. The aerosol preparation according to claim 21 or 22, wherein the at least one of the metering chamber, the slide member and the housing is made of resinous material having such a characteristic that, just after the resinous material having 39mm² surface area has been soaked in the 10ml aerosol composition having the 0.025wt% macrolide compound for a month under a condition of 75% relative humidity and 40°C, the amount of the remaining macrolide compound in the aerosol composition is not less than 80% of the amount of the initial macrolide compound before the soaking.

24. The aerosol preparation according to any of claims 1 to 23, the valve part further comprising a protection ring made of at least one resinous material selected from a plastic group including polyethylene, polybutylene terephthalate, polyacetal, polyamide, polytetrafluoroethylene and polypropylene.

25. The aerosol preparation according to claim 24, wherein the protection ring is made of polyethylene or polyamide.

26. The aerosol preparation according to claim 24 or 25, wherein the protection ring is made of resinous material having such a characteristic that, just after the resinous material having 39mm² surface area has been soaked in the 10ml aerosol composition having the 0.025wt% macrolide compound for a month under a condition of 75% relative humidity and 40°C , the amount of the remaining macrolide compound in the aerosol composition is not less than 80% of the amount of the initial macrolide compound before the soaking.
